(19) Europäisches Patentamt
European Patent Office
Office européen des brevets

(11) Veröffentlichungsnummer : **0 413 302 B1**

(12) # EUROPÄISCHE PATENTSCHRIFT

(45) Veröffentlichungstag der Patentschrift : **04.08.93 Patentblatt 93/31**

(51) Int. Cl.$^5$ : **C07D 265/06,** A61K 31/535

(21) Anmeldenummer : **90115558.0**

(22) Anmeldetag : **14.08.90**

(54) **Neue 1,3-Oxazine.**

(30) Priorität : **16.08.89 DE 3926898**

(43) Veröffentlichungstag der Anmeldung : **20.02.91 Patentblatt 91/08**

(45) Bekanntmachung des Hinweises auf die Patenterteilung : **04.08.93 Patentblatt 93/31**

(84) Benannte Vertragsstaaten : **AT BE CH DE DK ES FR GB GR IT LI LU NL SE**

(56) Entgegenhaltungen :
**DE-A- 2 221 408**
**DE-B- 1 146 068**
**US-A- 3 833 576**

(73) Patentinhaber : **Dr. Karl Thomae GmbH**
**Postfach 1755**
**W-7950 Biberach 1 (DE)**

(72) Erfinder : **Ballhause,Helmut**
**Fohrenweg 6**
**W-7950 Biberach 1 (DE)**
Erfinder : **Engelhardt,Günther,Prof.Dr.**
**Unterer Bühl 18**
**W-7950 Biberach 1 (DE)**
Erfinder : **Landgraf,Claus Adolf,Dr.**
**Schubertweg 3**
**W-7950 Biberach 1 (DE)**
Erfinder : **Mayer,Norbert,Dr.**
**Friedrich-Ebert-Strasse 66**
**W-7950 Biberach 1 (DE)**
Erfinder : **Roth,Willy,Dr.**
**Matthias-Erzberger-Str. 35**
**W-7950 Biberach 1 (DE)**
Erfinder : **Schumacher,Kurt,Dr.**
**Friedrich Profit-Strasse 9**
**W-6720 Speyer (DE)**
Erfinder : **Prox,Axel,Prof.Dr.**
**Schillerstrasse 24**
**W-7951 Warthausen (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Eines der wohl bekanntesten Hustenmittel ist das Morphinderivat "Codein" mit der Formel

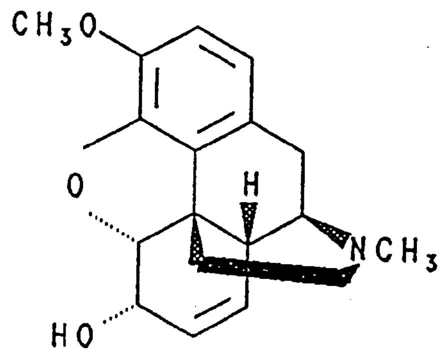

In der Deutschen Patentschrift P 1146068 werden basisch-substituierte Carbinole der allgemeinen Formel

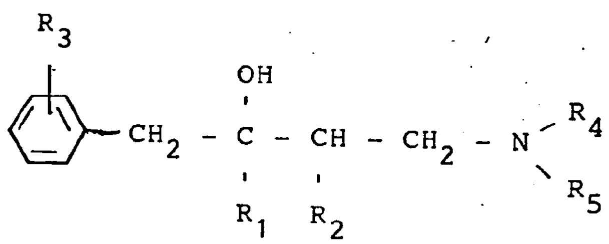

beschrieben, wobei $R_1$ bis $R_5$ niedrigmolekulare Alkylreste bedeuten und $R_3$ ein Wasserstoff- oder ein p-Halogenatom sein kann und die als Hustenmittel verwendet werden können. Eine dieser Verbindungen, 1-p-Chlorphenyl-2,3-dimethyl-4-dimethylaminobutanol, ist seit Jahren als Handelspräparat der Firma Dr. Karl Thomae GmbH unter dem Namen "Silomat" auf dem Markt. Die Substanz hat den INN generic name "Clobutinol".

Aufgabe der derzeitigen Erfindung ist, neue Verbindungen zu finden, die eine hustenstillende Wirkung, aber nicht die bekannten unangenehmen Nebenwirkungen von Codein, aufweisen.

Die Erfindung betrifft neue 1,3-Oxazine der allgemeinen Formel I

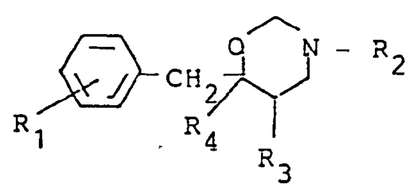

I.

in der $R_1$ ein Wasserstoff oder ein Halogenatom, $R_2$ eine $C_1$-$C_3$Alkylgruppe, und $R_3$ und $R_4$, die gleich oder verschieden sein können, $C_1$-$C_3$Alkylgruppen bedeuten, deren physiologisch verträgliche Säureadditionssalze mit anorganischen oder organischen Säure sowie Verfahren zu ihrer Herstellung.

Bevorzugte Verbindungen den obengenannten allgemeinen Formel I sind diejenigen, in denen $R_1$ ein Halogenatom in der p-Stellung ist.

Besonders bevorzugt ist die Verbindung, worin $R_1$ ein Cloratom ist, vorzugsweise p-Chlor, und $R_2$ bis $R_4$, die gleich oder verschieden sein können, Methyl oder Ethyl sind.

Das Verfahren zur Herstellung dieser neuen Verbindungen ist dadurch gekennzeichnet, daß man eine Verbindung der allgemeinen Formel II

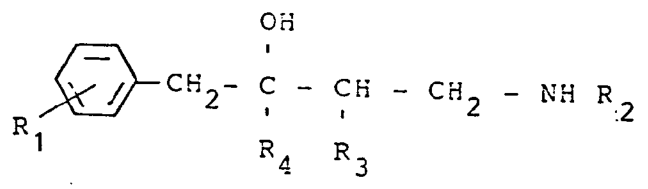 II.

oder deren Säureadditionssalze mit einer wässrigen Formaldehyd-Lösung umsetzt, zweckmäßig bei Temperaturen zwischen 20°C bis Siedetemperatur der Lösung, vorzugsweise jedoch bei Zimmertemperatur, die zunächst gebildete Verbindung der allgemeinen Formel mit an sich bekannten Methoden isoliert, vorzugsweise als Säureadditionssalz, und gewünschtenfalls nach an sich bekannten Methoden eine freie Base in ein Säureadditionssalz überführt oder ein Säureadditionssalz in eine freie Base überführt.

Die Verbindungen der allgemeinen Formel II können z.B. durch folgende Methoden hergestellt werden:

a) De-N-methylierung einer Verbindung der allgemeinen Formel III

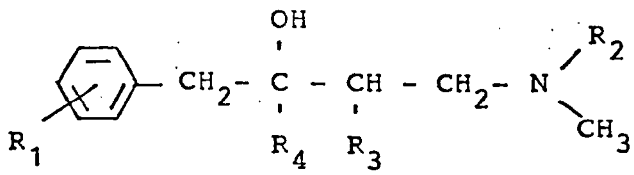 III.

oder deren Säureadditionssalze, wobei $R_1$ bis $R_4$ sind wie oben definiert sind, oder

b) Entfernung der Schutzgruppe Pg aus einer Verbindung der allgemeinen Formel IV

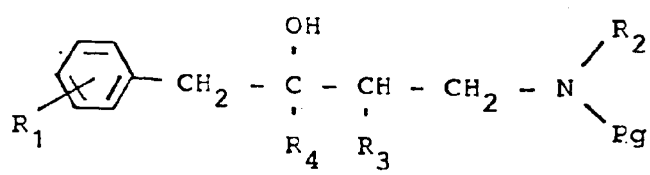 IV.

oder deren Säureadditionssalzen.

Die Mono-De-N-alkylierung nach Verfahren a) kann nach an sich bekannten Methoden durchgeführt werden, z.B. durch Umsetzung mit Azodicarbonsäure-diäthylester in einem unpolaren Lösungsmittel, wie Toluol, bei einer Temperatur bis zur Siede- temperatur des Ansatzes und Hydrolisierung der dabei ent- stehenden Produkte vorzugsweise unter Anwendung von Ammonium-Chlorid-Lösung in einem polaren Lösungsmittel wie Methanol-Wasser bei einer Temperatur bis zur Siedetemperatur des Ansatzes.

Verbindungen der allgemeinen Formel III sind literaturbekannt, wie z.B. aus den Deutschen Patentanmeldungen P 1146068 und P 1153380.

Das Verfahren b) kann nach an sich bekannten Methoden durchgeführt werden, abhängig von der Identität der Schutzgruppe. Vorzugsweise ist die Schutzgruppe die t-Butyloxycarbonylgruppe (Boc), die durch Trifluoressigsäure abgespalten werden könnte.

Die Verbindungen der allgemeinen Formel IV können nach an sich bekannten Methoden hergestellt werden. Vorzugsweise werden diese Zwischenprodukte unter Verwendung von Verbindungen der allgemeinen Formel III als Ausgangsstoff nach folgendem Reaktionsschema produziert:

Verbindungen der Formel III

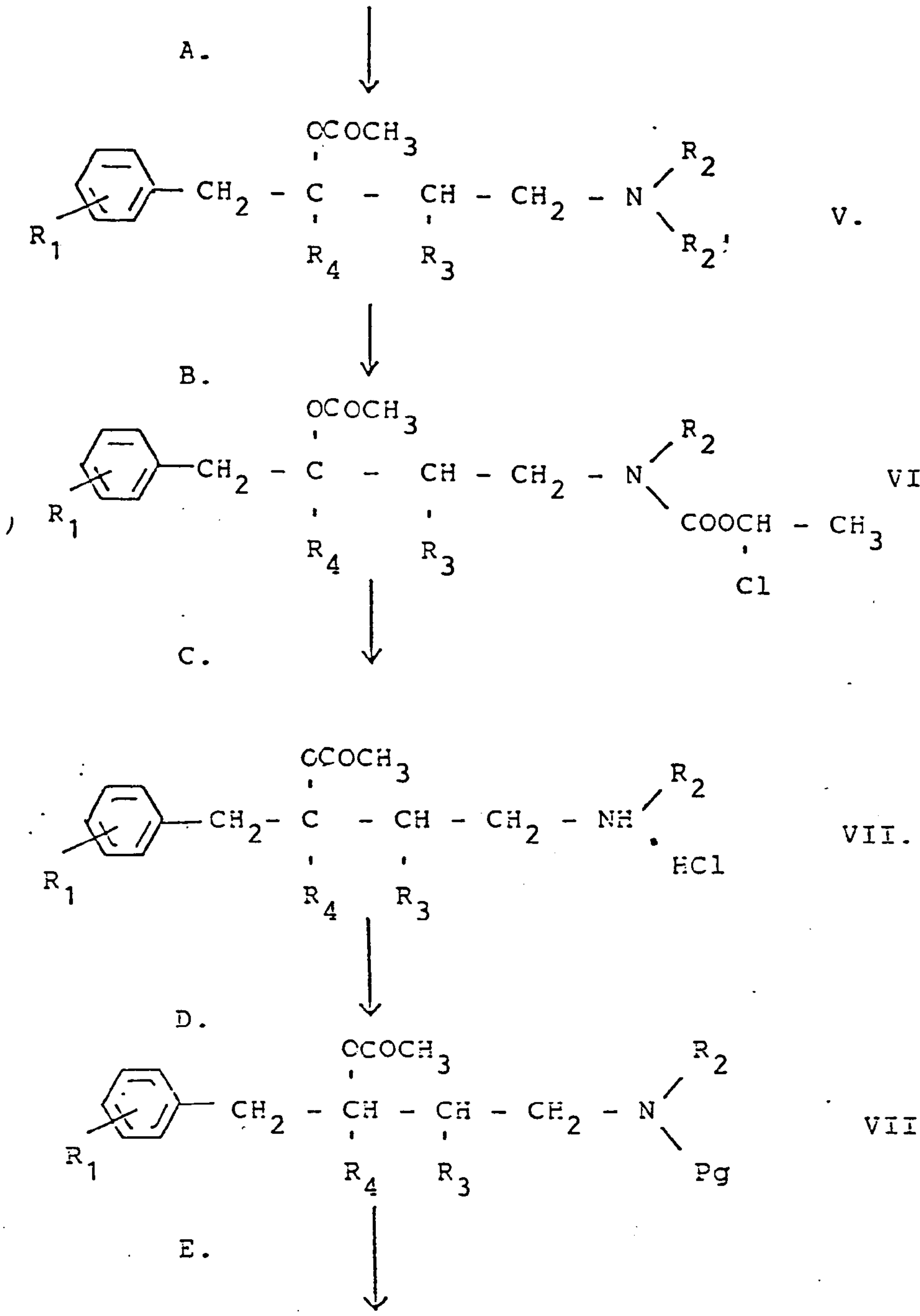


V.

VI.

VII.

VIII.

Verbindungen der Formel IV.

In der obigen Reaktionsreihe wird eine Verbindung der Formel III mit Acetylchlorid in einem inerten Lösungsmittel, z.B. Toluol, bei einer Temperatur bis zur Siedetemperatur umgesetzt. Die Verbindung V bzw. deren Hydrochloridsalz kann nach an sich bekannten Methoden isoliert werden, und dann mit Chlorameisensäure-α-Chloräthylester in 1,2-Dichloräthan oder einem anderen geeigneten organischen Lösungsmittel bei einer Temperatur bis zur Siedetemperatur des Ansatzes gerührt und die Verbindung VI nach an sich bekannten Nacharbeitungsmethoden isoliert werden. Danach wird die resultierende Verbindung in Methanol oder einem anderen inerten Lösungsmittel bis zur Siedetemperatur erhitzt. Das so hergestellte sekundäre Amin VII wird dann als Hydrochlorid ausgefällt, dieses wird isoliert und nach bekannten Methoden gereinigt und getrocknet. Die Verbindung VII wird dann nach an sich bekannten Methoden in geschützte Aminoderivate übergeführt. Vorzugsweise geschieht das mit Di-t-butyl-di-carbonat in einem organischen Lösungsmittel wie z.B. Dioxan in Anwesenheit von einer organischen Base wie z.B. Triethylamine bei einer Temperatur von 20°C bis zur Siedetemperatur des Ansatzes, vorzugsweise bei Zimmertemperatur.

Die erhaltenen Verbindungen der allgemeinen Formel I können, sofern sie nicht bereits bevorzugt als Säureadditionssalze hergestellt wurden, mit anorganischen oder organischen Säuren nach an sich bekannter Weise in ihre physiologisch verträglichen Säureadditionssalze überführt werden, so z.B. durch Umsetzung einer alkoholischen Lösung der Base mit der äquimolaren Menge der entsprechenden Säure in Äther. Als Säuren haben sich beispielsweise Salzsäure, Bromwasserstoffsäure, Schwefelsäure, Phosphorsäure, Milchsäure, Zitronensäure, Weinsäure oder Maleinsäure als geeignet erwiesen.

Die 1,3-Oxazine der Formel I stellen Racemate dar, diese können gegebenenfalls in ihre optisch aktiven Antipoden in üblicher Weise, z.B. mit optisch aktiven Säuren, durch fraktonierte Kristallisation aufgespalten werden. Die erfindungsgemäßen Verbindungen können aus z.B. den aktiven Antipoden der Verbindungen III hergestellt werden, wobei die Ausgangsstoffe z.B. der Formel III in ihre optisch aktiven Antipoden, z.B. durch flüssigchromatographische Racematetrennung, abgetrennt werden und die Verfahren a) oder b) durchgeführt werden. Die Zwischenprodukte V bis VIII können gleichfalls behandelt werden, sofern sie als Racemate vorkommen. Die optische Reinheit der Ausgangs- bzw. Zwischenstoffe z.B. der Formel III sowie der Antipoden der beanspruchten Verbindungen der Formel I kann flüssigchromatographisch an einer chiralen Säule (s. Abb. 1 bis 5) bewiesen werden.

Zur medizinischen Anwendung lassen sich die erfindungsgemäßen Verbindungen mit Hilfe von üblichen galenischen Hilfsstoffen wie z.B. Milchzucker, Mannit, Maisstärke, Methylcellulose, Hydroxyäthylcellulose, Polyäthylenoxid, hochdisperses Aluminiumoxid, Magnesium-Aluminiumsilikat, Magnesiumoxid, Magnesiumstearat, Natriumlaurylsulfat, Natriumcitrat, Weinsäure, Natriumpyrosulfit, Dioctylnatriumsulfosuccinat, p-Hydroxybenzoesäuremethylester-Natriumsalz, p-Hydroxybenzoesäurepropylester-Natriumsalz, Saccharin-Natrium, Aromastoffen und Entschäumer in die üblichen galenischen Zubereitungsformen wie Tabletten, Filmtabletten, Oblongtabletten, Dragées oder Kapseln sowie ihren Retardformen, Ampullen, Trockenampullen, Trockengranulaten oder Trockensäfte einarbeiten.

Die antitussive Wirkung der Verbindung 6-(p-Chlor-benzyl)-3,5,6-trimethyl-2H-3,4,5,6-tetrahydro-oxazin (1,3)-hydrochlorid wurde wie folgt geprüft:

33 männliche und weibliche Katzen mit einem Körpergewicht von 3,4 bis 4,5 kg wurden mit Pentobarbital-Na narkotisiert (45 mg/kg als initialer Bolus i.p. und anschließend über den Versuch verteilt nach Bedarf mehrfach 7,5 oder 15 mg/kg i.v.). Es wurde darauf geachtet, die Tiere etwa auf Stufe 1 bis 2 (Guedel) des Toleranzstadiums zu halten.

Die Auslösung der Hustenstöße erfolgte mechanisch durch Einführen eines Kunststoffkatheters mit kugeliger Spitze, Durchmesser ca. 1,5 mm, in die Trachea bis zur Bifurkation. Zu jedem Reizzeitpunkt wurde eine Serie von 3 Stimuli im gegenseitigen Abstand von etwa 30 Sekunden durchgeführt. Hustenstöße wurden immer 5, 25 und 45 Minuten nach Gabe der Prüfsubstanz bzw. des Vehikels provoziert.

Die Prüfsubstanz wurde als Lösung in 0,9%iger NaCl-Lösung durch einen in der V. femoralis liegenden Katheter appliziert. Das injizierte Volumen betrug maximal 0,5 ml/kg. Vor Substanzgabe wurde an jedem Tier der Effekt des Vehikels überprüft.

Zur Bewertung der hustendämpfenden Wirkung wurde die Anzahl der nicht mit Hustenstößen beantworteten Stimuli zu deren Gesamtzahl zu jedem Zeitpunkt nach Substanzgabe in Beziehung gesetzt und daraus die prozentuale Hemmung errechnet (Tabelle 1).

Tabelle 1

| Substanz | Dosis mg/kg i.v. | n | Prozentuale Hustenhemmung, MW ± SE nach Min. 5 | 25 | 45 |
|---|---|---|---|---|---|
| Kontrolle | - | 33 | 6,1 ± 3,4 | 5,1 ± 3,2 | 7,1 ± 3,3 |
| Wirkstoff | 0,5 | 5 | 26,7 ± 8,5 | 16,7 ± 10,5 | 23,3 ± 10,0 |
| | 0,705 | 5 | 23,3 ± 6,7 | 13,3 ± 6,2 | 13,3 ± 3,3 |
| | 1,0 | 5 | 33,3 ± 18,3 | 40,0 ± 16,3 | 36,7 ± 3,3 |
| | 1,41 | 5 | 63,3 ± 13,3 | 53,3 ± 16,2 | 50,0 ± 18,3 |
| | 2,0 | 5 | 33,3 ± 17,5 | 53,3 ± 14,3 | 36,7 ± 17,0 |
| | 2,82 | 5 | 36,7 ± 14,3 | 43,3 ± 13,5 | 40,0 ± 11,3 |
| | 4,0 | 5 | 60,0 ± 11,3 | 63,3 ± 13,3 | 46,7 ± 9,7 |
| | 5,64 | 5 | 70,0 ± 20,0 | 70,0 ± 20,0 | 63,3 ± 17,0 |
| | 8,0 | 3 | 44,4 ± 29,4 | 50,0 ± 25,5 | 44,4 ± 20,0 |

Mit Hilfe der linearen Regressionsanalyse und der linearen Kovarianzanalyse wurden nach Parallelanpassung $ED_{50}$-Wert (Reduktion der Anzahl der Hustenstöße um 50%) ermittelt (Tabelle 2).

Tabelle 2

| Minuten nach Substanzgabe | $ED_{50}$ (mg/kg i.v.) nach Parallelanpassung |
|---|---|
| 5 | 2,6 |
| 25 | 2,4 |
| 45 | 3,2 |

Die Substanz in den Dosierungen 0,5 bis 8 mg/kg i.v. verabreicht, wirkte vermindernd auf die Anzahl der mechanisch ausgelösten Hustenstöße. Hier war die hustenhemmende Wirkung von der niedrigsten verabreichten Dosierung ab zu allen Testzeiten zu beobachten.

Die erfindungsgemäßen neuen Verbindungen der allgemeinen Formel I und deren physiologisch verträglichen Säureadditionssalze eignen sich aufgrund ihrer oben erwähnten pharmakologischen Eigenschaften zum hustenstillenden Zweck insbesondere zur Behandlung des Hustens der Luftwege, des Pertussis, des Reiz- und Krampfhustens.

Die zur Erzielung einer entsprechenden Wirkung erforderliche Dosierung beträgt zweckmäßigerweise 2 bis 4 mal täglich 0,1 bis 4,0 mg/kg, vorzugsweise 0,3 bis 1,5 mg/kg Körpergewicht.

Beispiel 1

6-(p-Chlor-benzyl)-3,5,6-trimethyl-2H-3,4,5,6-tetrahydro-oxazin(1,3)-hydrochlorid

4,0 g 1-(p-Chlor-phenyl)-2-hydroxy-2,3-dimethyl-4-methylamino-butan-hydrochlorid werden in 10 ml Wasser gelöst und 10 ml ca. 36%ige Formaldehyd-Lösung hinzugefügt. Nach 12-stündigem Stehen bei Raumtemperatur wird der Ansatz unter Kühlung mit konz. Ammoniak alkalisiert und die Basenanteile ausgeäthert. Nach Trocknung der Ätherphase über Natriumsulfat wird das Filtrat eingedampft und der ölige Rückstand mit wenig Methanol aufgenommen. Die methanolische Lösung wird mit ätherischer Salzsäure auf einen pH von ca. 5 gebracht und mit so viel Äther versetzt, bis das entstandene Hydrochlorid ausfällt. Die Lösung wird so lange mit Essigester zum Sieden erhitzt, unter teilweisem Verdampfen von Äther und Methanol, bis das Hydrochlorid ausfällt. Das Hydrochlorid wird abgesaugt, mit Essigester ausgekocht, filtriert und getrocknet. Schmp. F = 198 - 200°C, Ausbeute: 3,3 g.

Behandelt man auf analoge Weise die Enantiomeren des nor-Clobutinols, so erhält man die Enantiomeren des beanspruchten 1,3-Oxazins mit folgenden Drehwerten:

Aus (+)-Clobutinol-> (+)-nor-Clobutinol-> (-)-1,3 Oxazin-HCl: $[\alpha]_{D(589)}^{20}$ = -16,83° (c=0,303; 1 dm; Äthanol rein)
Schmelzpunkt: 197-199°C

Aus (-)-Clobutinol-> (-)-nor Clobutinol-> (+)-1,3 Oxazin-HCl: $[\alpha]_{D(589)}^{20}$ = +16,6° (c=0,301; 1 dm; Äthanol rein)
Schmelzpunkt: 197-199°C

Beispiel 2

Tabletten mit 5.0 mg 6-(p-Chlor-benzyl)-3,5,6-trimethyl-2H-3,4,5,6-tetrahydro-oxazin (1,3)-hydrochlorid

Zusammensetzung:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemisch und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

Beispiel 3

Ampullen mit 10 mg 6-(p-Chlor-benzyl)-3,5,6-trimethyl-2H3,4,5,6-tetrahydro-oxazin (1,3)-hydrochlorid

Zusammensetzung:

| | | | |
|---|---|---|---|
| Wirkstoff | | 10,0 | mg |
| Natriumchlorid | | 8,0 | mg |
| Dest. Wasser | ad | 1 | ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120°C.

Beispiel 4

Tropfen mit 0,5 mg 6-(p-Chlor-benzyl)-3,5,6-trimethyl-2H-3,4,5,6-tetrahydro-oxazin (1,3)-hydrochlorid pro 100.0 ml

Zusammensetzung:

| | | | |
|---|---|---|---|
| P-Hydroxybenzoesäuremethylester | | 0,035 | g |
| p-Hydroxybenzoesäurepropylester | | 0,015 | g |
| Anisöl | | 0,05 | g |
| Menthol | | 0,06 | g |
| Ethanol rein | | 10,0 | g |
| Wirkstoff | | 0,5 | g |
| Natriumcyclamat | | 1,0 | g |
| Glycerin | | 15,0 | g |
| Dest. Wasser | ad | 100,0 | ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser aus 100 ml aufgeführt und schwebeteilchenfrei filtriert.

REFERENZ-BEISPIEL 1

1-(p-Chlor-phenyl)-2-hydroxy-2,3-dimethyl-4-methylamino-butan-hydrochlorid

26,5 g Clobutinol-Base werden mit 21,0 g Azodicarbonsäurediäthylester (1,1 molar zu Clobutinol) in 200 ml trockenem Toluol 4 Stunden zum Sieden erhitzt. Nach Abdampfen des Toluols im Vakuum wird der Rückstand 4 Stunden lang bei Siedetemperatur mit 200 ml Methanol und 200 ml gesättigter Ammoniumchlorid-Lösung hydrolisiert.

Der Ansatz wird eingedampft und der resultierende halbfeste Rückstand mit 100 ml eines Gemisches gleicher Anteile konz. Salzsäure und Wasser versetzt. Nach dem Absaugen über einen Weitlauffilter und erneutem Filtrieren des Filtrats wird das Filtrat mit 40%iger Natronlage und Eis alkalisiert, und die basischen Anteile werden mit Äther ausgeschüttelt. Die Ätherphase wird abgetrennt und über Natriumsulfat getrocknet, danach mit Aktivkohle ausgerührt, filtriert und eingedampft. Der eingedampfte Rückstand wird säulenchromatographisch an Kieselgel (0,05 - 0,2 mm) mit dem Eluanten Methylenchlorid - Methanol - Ammoniak (940+60+4) bis (900+100+6) gereinigt. Die polaren Frakionen, die das gewünschte nor-Clobutinol enthalten, werden vereinigt und das Lösungsmittel abdestilliert. Die Rohbase wird in das Hydrochlorid überführt. Den basischen Rückstand nimmt man in wenig Methanol auf und neutralisiert mit ätherischer Salzsäure. Dabei fällt das Hydrochlorid der

gewünschten Verbindung aus. Den Niederschlag vervollständigt man durch weitere Ätherzugabe.
Schmp. F = 182 - 183°C, ca. 4,0 g.

Ausgehend von (+)- bzw. (-)-Clobutinol erhält man das entsprechende enantiomere nor-Clobutinol.

REFERENZ-BEISPIEL 2

1-(p-Chlor-phenyl)-2-hydroxy-2,3-dimethyl-4-methylamino-butan hydrochlorid

A. O-Acetyl-Clobutinol

30,0 g Clobutinol-Base in 150 ml Toluol werden tropfenweise bei 80°C Ölbadtemperatur mit 8,5 ml Acetylchlorid in 20 ml Toluol versetzt und anschließend das Reaktionsgemisch 2 Stunden lang zum Sieden erhitzt. Die schnell einsetzende Ausfällung des gewünschten Hydrochlorids wird nach dem Abkühlen des Ansatzes abgesaugt, mit Äther nachgespült und getrocknet.

B. N-(α-chloräthyl-Carbamat) des O-Acetyl-nor-Clobutinols

16,0 g O-Acetyl-Clobutinol-Base (Stufe A) in 100 ml 1,2-Dichloräthan werden mit 11,0 g Chlorameisensäure-α-chloräthylester in 50 ml 1,2-Dichloräthan 3 Stunden zum Sieden erhitzt. Dann wird der Ansatz mit 1N-Salzsäure und Eis ausgeschüttelt, die organische Phase abgetrennt und neutral mit Kaliumbicarbonat-Lösung (versetzt mit Eis) ausgeschüttelt. Die organische Schicht wird abgetrennt, über Natriumsulfat getrocknet und die Lösung abfiltriert und zur Trockne eingedampft.

Ohne weitere Reinigung wird der Rückstand weiter verarbeitet.

C. O-Acetyl-nor-Clobutinol-hydrochlorid

Der ölige Rückstand der vorstehenden Verbindung aus Stufe B wird mit 50 ml Methanol aufgenommen und 2 Stunden am Rückfluß erhitzt. Dabei beginnt das gewünschte Hydrochlorid bereits auszufallen. Ein Teil des Methanols wird dann noch abdestilliert, der Ansatz gekühlt und das Kristallisat abgesaugt. Ca. 10,0 g Hydrochlorid fallen nach Spülung mit Essigester und Trockne an.
F = 185 - 188°C

D. O-Acetyl-N-Boc-nor-Clobutinol

12,8 g des Produktes aus Stufe C. werden in 500 ml Dioxan zusammen mit 10,0 g Di-t-butyl-di-carbonat und 4,0 g Triäthylamin 12 Stunden bei Raumtemperatur gerührt. Dann wird die Suspension abgesaugt, filtriert und das Filtrat eingeengt. Der Rückstand wird ohne weitere Reinigung weiterverarbeitet.

E. N-Boc-nor-Clobutinol

Obiger öliger Rückstand aus Stufe D wird mit 50 ml 1N-Natronlauge und 100 ml Methanol 3 Stunden am Rückfluß erhitzt. Danach wird das Methanol im Vakuum abgedampft und die alkalische, wässrige Suspension ausgeäthert. Die Äther-Phase wird abgetrennt, über Natriumsulfat getrocknet und nach Filtration eingedampft. Der zunächst ölige Rückstand wird nach einiger Zeit fest. Der feste Rückstand wird gepulvert und direkt der Umsetzung mit Trifluoressigsäure unterworfen.

F. nor-Clobutinol

12,0 g der vorstehenden Boc-Verbindung aus Stufe E werden in 40 ml eisgekühlter Trifluoressigsäure gelöst. Die Reaktionslösung wird 30 Minuten bei Eiskühlung und 2 Stunden bei Raumtemperatur stehen gelassen. Sodann wird der größte Teil der Trifluoressigsäure bei 30°C abdestilliert, der Rückstand in Wasser und Eis aufgenommen und die nichtbasischen Anteile mit Äther ausgeschüttelt. Die wässrige Phase wird abgetrennt, unter Eiskühlung mit 40%iger Natronlauge alkalisiert und mit Methylenchlorid ausgeschüttelt. Die organische Phase wird abgetrennt und abdestilliert und der Rückstand in das Hydrochlorid überführt. Dazu wird die ölige Base in wenig Methanol gelöst und ätherische Salzsäure bis zum pH 5 hinzugefügt. Das Hydrochlorid fällt dabei schon aus. Der Niederschlag wird durch weitere Zugabe von Äther vervollständigt. Das abgesaugte Hydrochlorid wäscht man mit Essigsäureäthylester aus.
Nach Trocknung zeigt das Salz einen

Schmp. von F = 182 - 184°C, Ausbeute: 8,0 g

REFERENZ-BEISPIEL 3

Analytischer Nachweis der optischen Einheit der I- und der II-Enantiomere

(A) Das Racemat(±) 1-p-Chlorphenyl-2,3-dimethyl-4-dimethylamino-butan-2-ol wurde auf eine LKB-Säule gegeben und mittels Propanol-2 (0,5%) und Phosphatbuffer pH 6 eluiert. Wie in Abbildung 1 dargestellt, wurde das (-)Enantiomer bei 9.25 Minuten und das (+)Enantiomer bei 12.25 Minuten erhalten.
(B) Das Racemat(±) 6-(p-Chlorbenzyl)-3,4,5-trimethyl-2H-3,5,6-tetrahydrooxazin(1,3) wurde auf eine Chiracel OD-Säule gegeben und mittels 495 ml Hexan und 5 ml Propanol-2 eluiert. Wie in Abbildung 2 dargestellt, wurde der (-)Enantiomer bei 11.89 Minuten und das (+)Enantiomer bei 13.22 Minuten erhalten.
(C) Die (+) und (-)Enantiomere der Verbindung 6-(p-Chlorbenzyl)-3,5,6-trimethyl(-2H-3,4,5,6-tetrahydro-oxazin(1,3) wurden durch stereospezifische Verfahren mittels des entsprechenden (+) und (-)Clobutinol als Ausgangsstoff hergestellt. Abbildung 3 und 4 stellen die Ergebnisse der chromatographischen Analyse der entsprechenden Produkte auf einer Chiracel 0D-Säule dar. Die (+) und (-)Enantiomere des Oxazin wurden gemischt und gleichfalls eluiert, das Ergebnis ist in Abbildung 5 dargestellt.

REFERENZ-BEISPIEL 4

Bindungsversuch an den Opiatrezeptor

In jedem Versuch wurden zwei männliche Ratten (ca. 200 g) mittels Schlag an den Hals getötet. Die Gehirne wurden entfernt und jeweils der Gehirnstamm zusammen mit der Medulla vorbereitet und gewogen. Diese Gewebe wurden mittels einem "Potter homogenizer" in 30 ml 50 nM Tris HCl Puffer pH 7.4 homogenisiert. Das resultierende Homogensat wurde bei 18.000 x g 15 Minuten lang zentrifugiert.

Durch nachfolgende Resuspensierung und Zentrifugierung wurden die Pellets zweimal gewaschen. Die resultierenden Pellets wurden mit dem 200-fachen ihres Gewichts an Tris HCl Buffer pH 7.4 verdaut. Zur Durchführung der Bindungsanpassung wurden jeweils 1 ml dieses Präparates mit 0.5mM 3H-Diprenorphine (38,9 Ci/nmol Amersham), das ein nicht-selektiver Ligand für die Opiatrezeptoren ist, und die Testverbindung bei verschiedenen Konzentrationen in einem Eisbad inkubiert.

Die Inkubation wurde nach 3 Stunden beendet und der Ansatz in einem "Ismatec Filter-Prep 101 sample processor" unter Verwendung eines Whatman GF/B Glaswollfilters schnell filtriert.

Das Filtrat wurde dreimal mit 3 ml gekohltem Buffer gespült und in kleinen Ampullen, die 4 ml Instasel enthielten, abgefüllt, über Nacht extrahiert. Danach wurde die Radioaktivit gemessen. Alle Messungen wurden dreimal durchgeführt. Als nicht-spezifische Bindung gilt jene gebundene Radioaktivität, die in Anwesenheit von 100 nM Naloxone gefunden wurde.

Ergebnisse:

Die radiomarkierte opiatagonistische Verbindung 3H-Diprenorphine bindet spezifisch und reversibel mit den Opiatrezeptoren der Präparate. Nach Scatchard Analysierung wurde bei einer Sättigungsuntersuchung ein $K_D$-Wert von 0.51 nM kalkuliert. Die spezifische Bindung von 0.5 nM 3H-Diprenorphine würde durch Naloxone mit einem $IC_{50}$Wert von 3 nM inhibiert. Im Gegensatz zu diesem Befund weist die Testverbindung bis 100 µM keine Inhibierung von 3H-Diprenorphine auf.

**Patentansprüche**

**1.** Verbindungen der allgemeinen Formel I

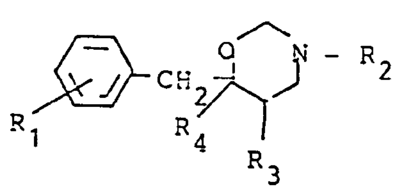

I.

in der $R_1$ ein Wasserstoff- oder ein Halogenatom, $R_2$ eine $C_1$-$C_3$Alkylgruppe, und $R_3$ und $R_4$, die gleich oder verschieden sein können, $C_1$-$C_3$Alkylgruppen bedeuten, und deren physiologisch verträgliche Säureadditionssalze mit anorganischen oder organischen Säuren.

2. Verbindungen gemäß Anspruch 1, worin $R_1$ ein Halogenatom in der p-Stellung bedeutet.

3. Verbindungen gemäß Anspruch 1 oder 2, worin $R_1$ ein Chloratom und $R_2$, $R_3$ und $R_4$, die gleich oder verschieden sein können, Methyl- oder Ethyl bedeuten.

4. 6-(p-Chlor-benzyl)-3,5,6-trimethyl-2H-3,4,5,6-tetrahydro-oxazin(1,3)- oder seine physiologisch verträglichen Säureadditionssalze mit anorganischen oder organischen Säuren, vorzugsweise Salzsäure.

5. Verwendung einer Verbindung gemäß einer der Ansprüche 1 bis 4 zur Herstellung eines Arzneimittels zur Behandlung von Husten.

6. Arzneimittel, enthaltend als Wirkstoff eine Verbindung gemäß den Ansprüchen 1 bis 4 neben gegebenenfalls einem oder mehreren inerten Trägerstoffen und/oder Verdünnungsmitteln.

7. Verfahren zur Herstellung einer Verbindung gemäß den Ansprüchen 1 bis 4, dadurch gekennzeichnet, daß man eine Verbindung der allgemeinen Formel II

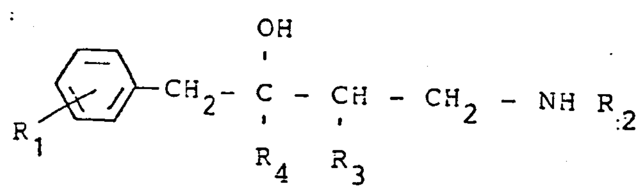

II.

in der $R_1$, $R_2$, $R_3$ und $R_4$ wie in Anspruch 1 definiert sind, oder deren Säureadditionssalze, mit einer Formaldehyl-Lösung umsetzt, zweckmäßigerweise bei Temperaturen zwischen 20°C bis Siedetemperatur der Lösung, vorzugsweise bei Zimmertemperatur, und die resultierende Verbindung gemäß Formel I des Anspruchs 1 als Säureadditionssalze oder freie Basen isoliert und gewünschtenfalls eine freie Base in ein Säureadditionssalz überführt oder ein Säureadditionssalz in eine freie Base überführt.

## Claims

1. Compounds of general formula I

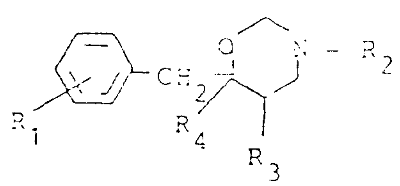

I

wherein $R_1$ denotes a hydrogen or halogen atom, $R_2$ denotes a $C_{1-3}$-alkyl group and $R_3$ and $R_4$, which may be identical or different, denote $C_{1-3}$-alkyl groups, and the physiologically acceptable acid addition salts thereof with organic or inorganic acids.

2. Compounds according to claim 1, wherein $R_1$ denotes a halogen atom in the p-position.

3. Compounds according to claim 1 or 2, wherein $R_1$ denotes a chlorine atom and $R_2$, $R_3$ and $R_4$, which may be identical or different, denote methyl or ethyl.

4. 6-(p-Chloro-benzyl)-3,5,6-trimethyl-2H-3,4,5,6-tetrahydro-oxazin(1,3)- or the physiologically acceptable acid addition salts thereof with organic or inorganic acids, preferably hydrochloric acid.

5. Use of a compound according to one of claims 1 to 4 for preparing a pharmaceutical composition for treating coughs.

6. Pharmaceutical composition containing as active substance a compound according to claims 1 to 4 optionally together with one or more inert carriers and/or diluents.

7. Process for preparing a compound according to claims 1 to 4, characterised in that a compound of general formula II

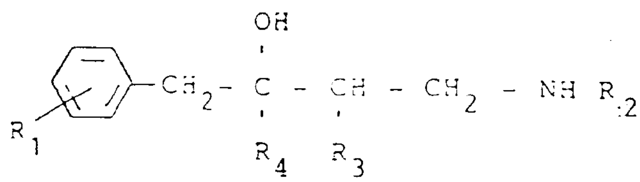

II

wherein $R_1$, $R_2$, $R_3$ and $R_4$ are defined as in claim 1, or an acid addition salt thereof, is reacted with a formaldehyde solution, expediently at temperatures between 20°C and the boiling temperature of the solution, preferably at room temperature, and the resulting compound according to formula I of claim 1 is isolated in the form of an acid addition salt or a free base and, if desired, a free base is converted into an acid addition salt or an acid addition salt is converted into a free base.

## Revendications

1. Composés de formule générale I

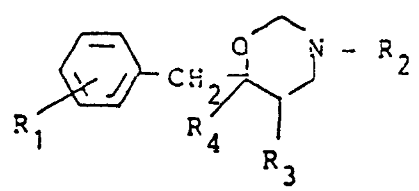

I.

dans laquelle $R_1$ représente un atome d'hydrogène ou d'halogène, $R_2$ représente un groupe allyle en $C_1$-$C_3$, et $R_3$ et $R_4$, qui peuvent être identiques ou différents, représentent des groupes allyle en $C_1$-$C_3$, et leurs sels d'addition d'acide acceptables du point de vue physiologique avec des acides inorganiques ou organiques.

2. Composés selon la revendication 1, dans lesquels $R_1$ représente un atome d'halogène en position p.

3. Composés selon la revendication 1 ou 2, dans lesquels $R_1$ représente un atome de chlore et $R_2$, $R_3$ et $R_4$, qui peuvent être identiques ou différents, représentent des groupes méthyle ou éthyle.

4. 6-(p-chlorobenzyl)-3,5,6-triméthyl-2H-3,4,5,6-tétrahydro-oxazine(1,3) ou ses sels d'addition d'acide acceptables du point de vue physiologique avec des acides inorganiques ou organiques, de préférence l'acide chlorhydrique.

5. Utilisation d'un composé selon l'une des revendications 1 à 4 pour la préparation d'un médicament pour le traitement de la toux.

6. Médicament contenant comme principe actif un composé selon les revendications 1 à 4 et éventuellement un ou plusieurs véhicules et/ou diluants inertes.

7. Procédé de préparation d'un composé selon les revendications 1 à 4, caractérisé en ce que l'on fait réagir un composé de formule générale II

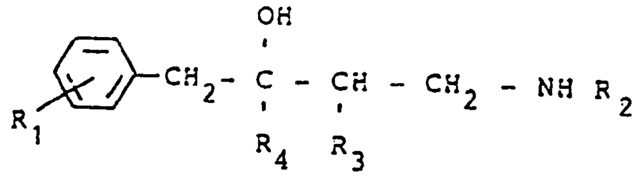

II.

dans laquelle $R_1$, $R_2$, $R_3$ et $R_4$ sont définis comme dans la revendication 1, ou ses sels d'addition d'acide, avec une solution de formaldéhyde, de manière judicieuse à des températures comprises entre 20°C et la température d'ébullition de la solution, de préférence à la température ambiante, et l'on isole le composé selon la formule I de la revendication 1 résultant sous forme de sels d'addition d'acide ou de bases libres et, si on le souhaite, on convertit une base libre en un sel d'addition d'acide ou un sel d'addition d'acide en une base libre.

Flüssigchromatographische Racematentrennung

Abbildung 1

(±)-Clobutinol

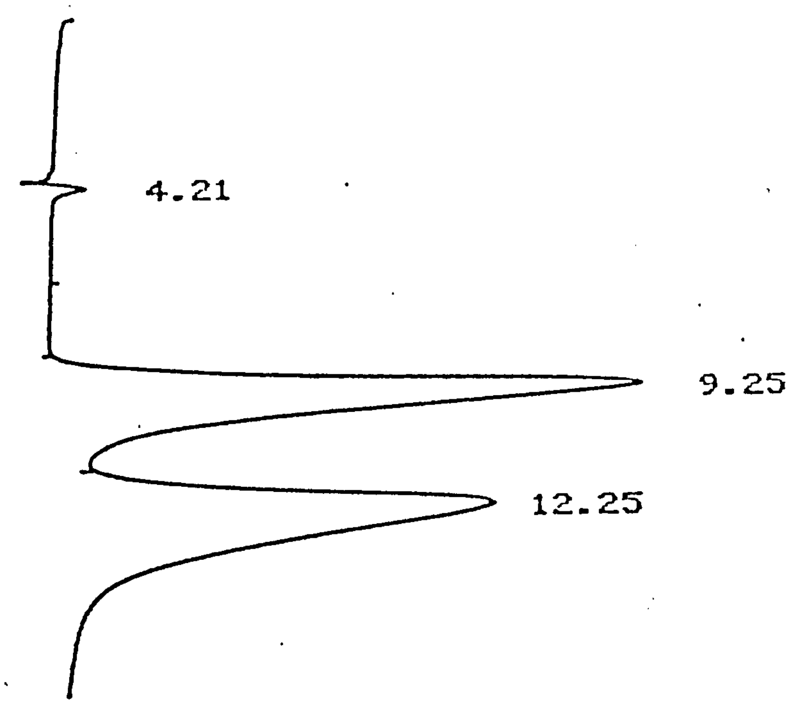


Säule: Enantiopac LKB

Eluant: Phosphatpuffer $p_H$=6 und Propanol-2 (0,5 %)

Abbildung 2

(±) 6-(p-Chlorbenzyl)-3,5,6-trimethyl-2H-3,4,5,6-tetrahydro-oxazin(1,3)

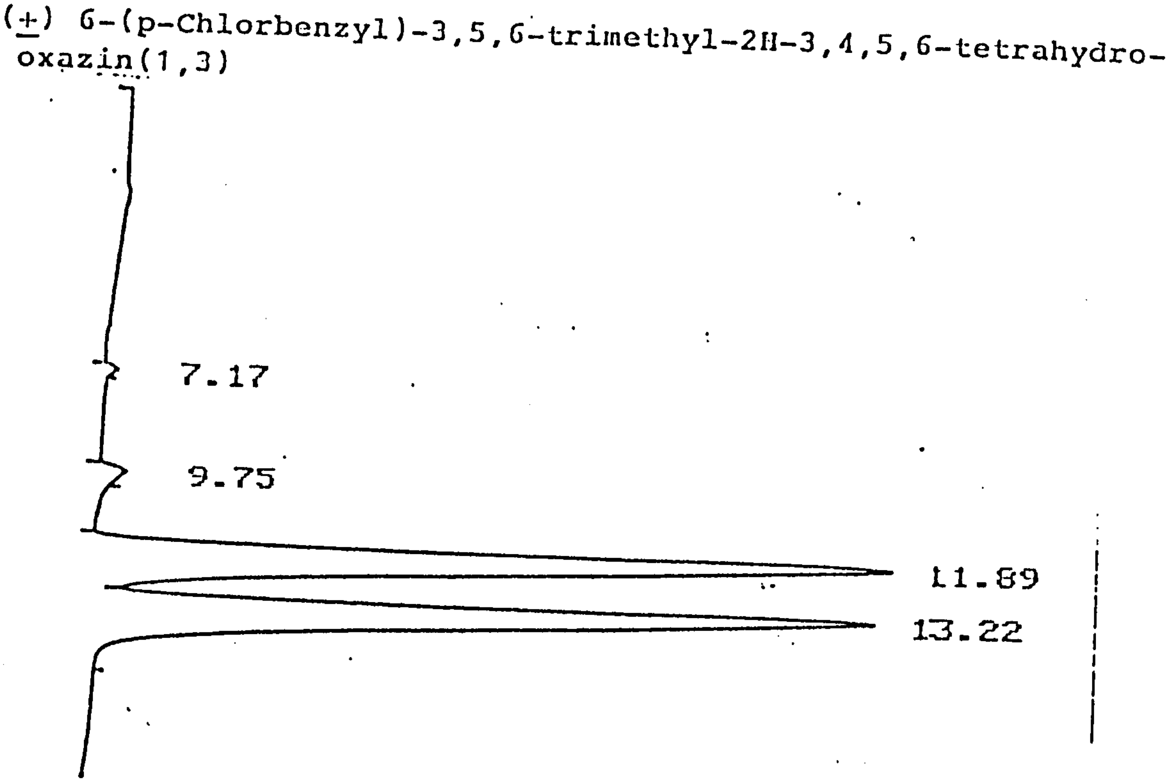


Säule: Chiralcel OD

Eluant: 495 ml Hexan und 5 ml Propanol-2

Abbildung 3

(-)-6-(p-Chlorbenzyl)-3,5,6-trimethyl-2H-3,4,5,6-tetrahydro-oxazin(1,3)

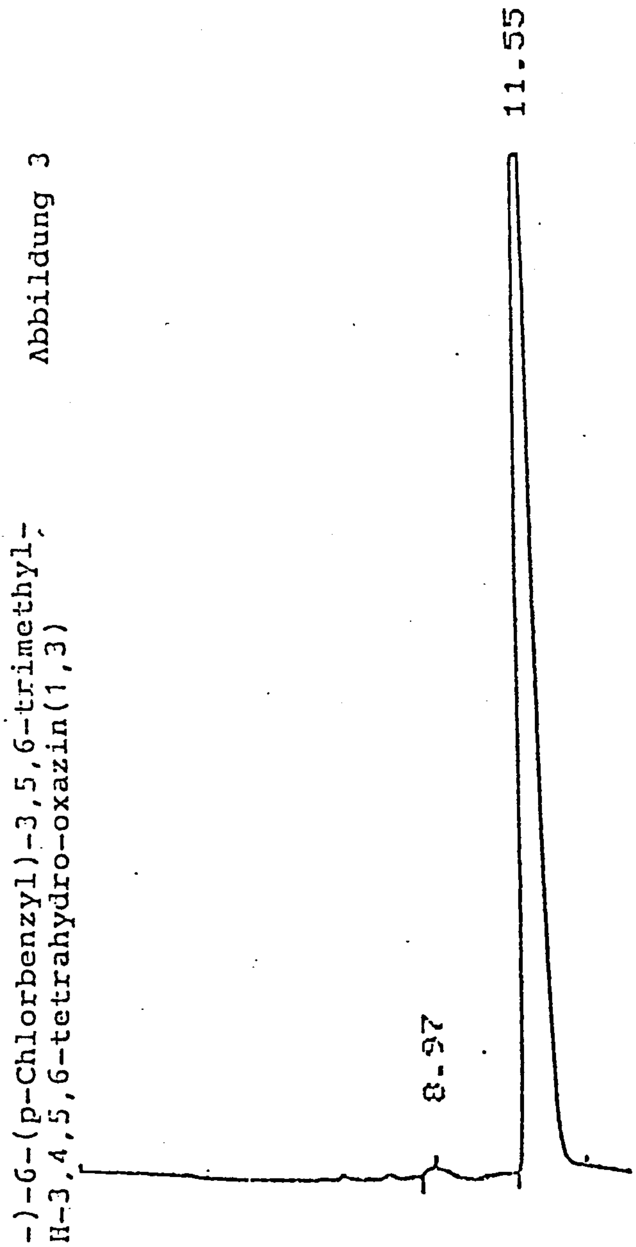


LC-Bedingungen wie bei Abb. 2

(+)-6-(p-Chlorbenzyl)-3,5,6-trimethyl-2H-3,4,5,6-tetrahydro-oxazin(1,3)

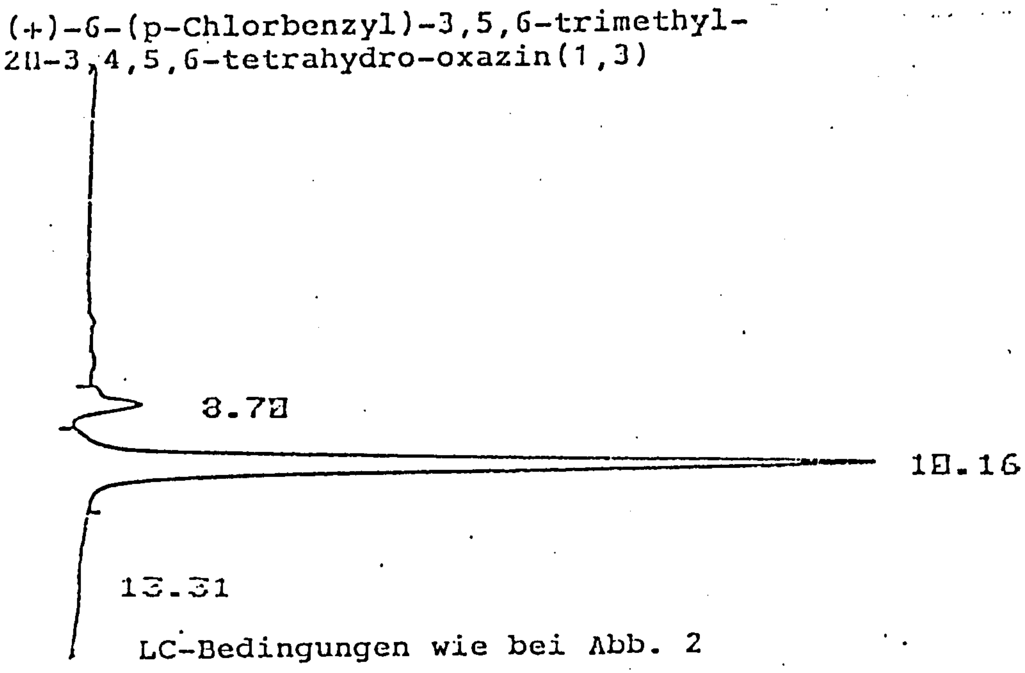


LC-Bedingungen wie bei Abb. 2

Abbildung 4

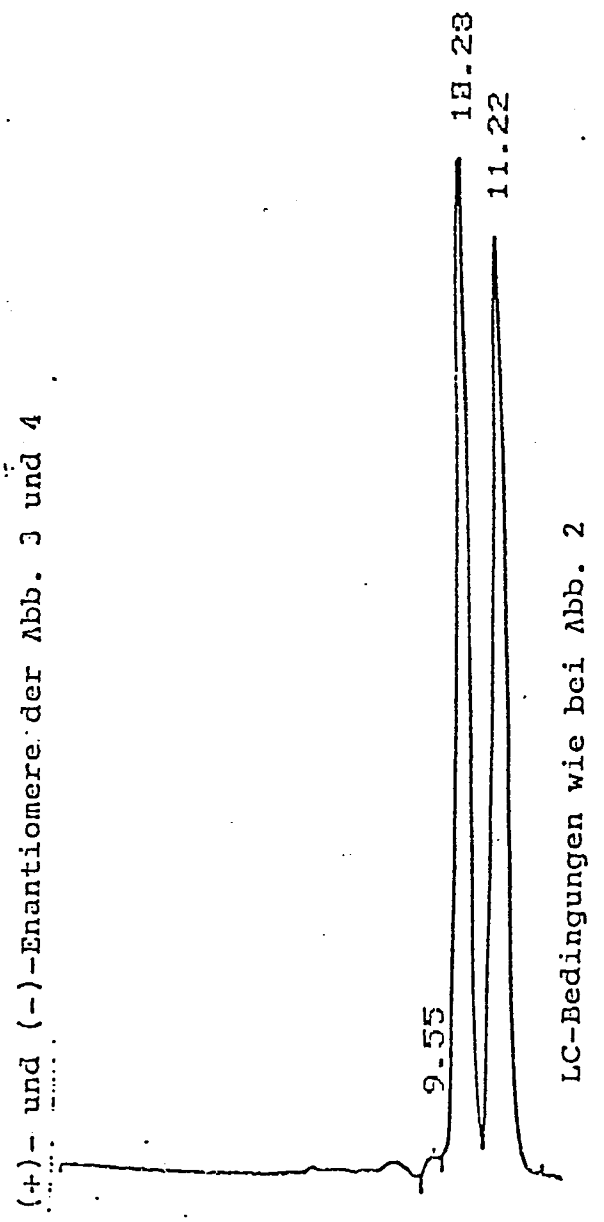
(+)- und (-)-Enantiomere der Abb. 3 und 4
9.55
10.23
11.22
LC-Bedingungen wie bei Abb. 2

Abbildung 5